(19) Europäisches Patentamt
European Patent Office
Office européen des brevets

(11) Veröffentlichungsnummer : **0 467 838 A2**

(12) # EUROPÄISCHE PATENTANMELDUNG

(21) Anmeldenummer : **91810542.0**

(51) Int. Cl.$^5$ : **A61K 9/127**

(22) Anmeldetag : **09.07.91**

(30) Priorität : **17.07.90 CH 2371/90**

(43) Veröffentlichungstag der Anmeldung :
**22.01.92 Patentblatt 92/04**

(84) Benannte Vertragsstaaten :
**AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Van Hoogevest, Peter, Dr.**
**Burgstrasse 5**
**CH-4125 Riehen (CH)**
Erfinder : **Isele, Ute**
**Rheinstrasse 19**
**W-7841 Bad Bellingen (DE)**

(54) **Verfahren zur Herstellung einer injizierbaren Liposomendispersion.**

(57) Die vorliegende Erfindung betrifft ein neues, vorteilhaftes Verfahren zur Herstellung einer injizierbaren Liposomendispersion. Man löst die Phospholipidkomponenten sowie den zu applizierenden Wirkstoff in konzentrierter Essigsäure, neutralisiert und dispergiert die Injektionslösung, welche direkt applizierbar ist.

EP 0 467 838 A2

Die vorliegende Erfindung betrifft ein neues, vorteilhaftes Verfahren zur Herstellung einer injizierbaren Liposomendispersion. Diese ist in erster Linie für die intravenöse Applikation verwendbar.

Injizierbare Liposomendispersionen mit verschiedenen Wirkstoffen und Phospholipiden wie Lecithin und Phosphatidylserin als Hilfsstoffen sind in zahlreichen Publikationen beschrieben und bereits klinisch erprobt worden. Zur Illustration des Standes der Technik wird die Europäische Patentanmeldung 178 624 genannt, worin eine Liposomendispersion mit synthetischem, gereinigtem Natrium-1,2-di-(9-cis-octadecenoyl)-3-sn-phosphatidyl-S-serin und 1-n-Hexadecanoyl-2-(9-cis-octadecenoyl)-3-sn-phosphatidylcholin als Phospholipiden und N-Acetyl-D-muramyl-L-alanyl-D-isoglutaminyl-L-alanin-2-(1,2-dipalmitoyl-sn-glycero-3-hydroxyphosphoryloxy)-äthylamid als verkapseltem Wirkstoff beschrieben ist. Diese Dispersion ist u.a. intravenös applizierbar.

Die injizierbaren Liposomendispersionen sind jedoch nachteilig, da sie als Vorstufe die Herstellung eines Trockenpräparats, z.B. Lyophilisat oder Filmrückstand, voraussetzen, das man vor der Applikation *in-situ* zur Bildung von Liposomen in Wasser dispergiert.

Gemäss U.S. Patentschrift 4,687,661 lassen sich auch ohne die Vorstufe des Trockenpräparats Liposomendispersionen durch Auflösen der Lipidkomponenten in ausgewählten, nicht-flüchtigen organischen Lösungsmitteln wie Glycerin oder Äthylenglycol und Erwärmen und Dispergieren der organischen Lösung in Wasser herstellen. Wegen des hohen Anteils an organischen Lösungsmitteln und möglicher thermischer Abbauprodukte sind diese Lösungen für Injektionslösungen insbesondere für die intravenöse Applikation physiologisch bedenklich.

Der vorliegenden Erfindung liegt die Aufgabe zugrunde, durch Wahl eines pharmazeutisch geeigneten, nicht-toxischen Lösungsmittels unter Umgehung der Vorstufe des Trockenpräparats durch Vermischen der Komponenten eine injizierbare Liposomendispersion herzustellen. Diese Aufgabe wird durch die vorliegende Erfindung gelöst, welche ein neues, vorteilhaftes Verfahren zur Herstellung einer injizierbaren Liposomendispersion zum Gegenstand hat.

Die Erfindung betrifft ein Verfahren zur Herstellung einer injizierbaren Liposomendispersion enthaltend

a) ein Phospholipid der Formel

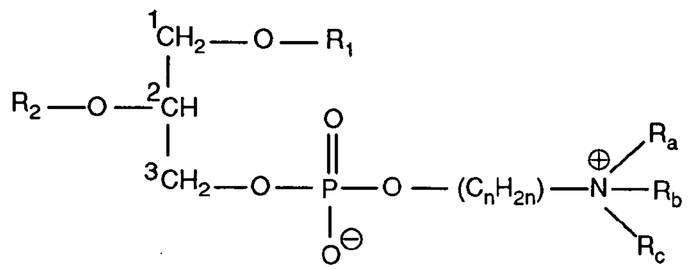

(I),

worin $R_1$ $C_{10-20}$-Acyl, $R_2$ Wasserstoff oder $C_{10-20}$-Acyl, $R_a$, $R_b$ und $R_c$ Wasserstoff oder $C_{1-4}$-Alkyl und n eine ganze Zahl von zwei bis vier darstellen, gegebenenfalls kombiniert mit einem weiteren

b) Phospholipid der Formel

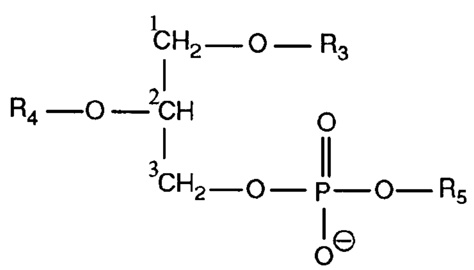

(II),

worin $R_3$ $C_{10-20}$-Acyl, $R_4$ Wasserstoff oder $C_{10-20}$-Acyl und $R_5$ Wasserstoff, $C_{1-4}$-Alkyl, $C_{1-5}$-Alkyl substituiert durch Carboxy, $C_{2-5}$-Alkyl substituiert durch Hydroxy, $C_{2-5}$-Alkyl substituiert durch Carboxy und Hydroxy oder $C_{2-5}$-Alkyl substituiert durch Carboxy und Amino,

c) den zu injizierenden Wirkstoff oder eine Wirkstoffkombination und

d) eine pharmazeutisch annehmbare Trägerflüssigkeit und gegebenenfalls weitere für Injektionszubereitungen geeignete Hilfsstoffe.

Das Verfahren ist dadurch gekennzeichnet, dass man eine Lösung oder Suspension der Komponenten a) und c) oder a), b) und c) in konzentrierter Essigsäure in der Trägerflüssigkeit d) dispergiert und die erhältliche Dispersion auf einen physiologisch annehmbaren pH-Wert bringt und gegebenenfalls für Injektionszubereitungen geeignete Hilfsstoffe zusetzt und gegebenenfalls eine Fraktion von Liposomen mit einem gewünschten Bereich des Durchmessers abtrennt

Die nach diesem Verfahren herstellbare vorteilhafte Liposomendispersion ist frei von Feststoffpartikeln und grösseren Lipidaggregaten, bei Zimmertemperatur mindestens mehrere Stunden stabil, reproduzierbar bezüglich des Mengenanteils der komponenten, toxikologisch unbedenklich und daher insbesondere für die intravenöse Applikation am Menschen geeignet.

Das Verfahren hat insbesondere den Vorteil, dass das organische Lösungsmittel Essigsäure nach dem Dispergieren durch Zugabe einer verdünnten wässrigen Base, wie verdünnter Natriumhydroxidlösung, in das physiologisch unbedenkliche Acetatsalz überführbar ist. Bei vollständiger Neutralisation ist daher die Injektionslösung frei von organischen Lösungsmitteln und für die intravenöse Applikation unproblematisch. Es entfällt der aufwendige Schritt, das organische Lösungsmittel aus der Dispersion zu entfernen.

Die weiter vorn und im folgenden genannten Begriffe werden im Rahmen der Beschreibung der Erfindung vorzugsweise wie folgt definiert:

Eine injizierbare Liposomendispersion ist parenteral, vorzugsweise intravenös, aber auch intramuskulär, z.B. zur Bildung eines Depots, oder subkutan, z.B. zur Applikation von Lokalanästhetika, verabreichbar.

Die injizierbare Liposomendispersion enthält Liposomen in Form von unilamellaren, vorzugsweise multilamellaren, grossen und kleinen Liposomen bestehend aus einer Doppelschichtanordnung der Phospholipide der Formel I und gegebenenfalls der Formel II mit einem Innenraum und sphärischer Gestalt (unilamellar) oder bestehend aus mehreren konzentrischen Doppelschichtanordnungen der Phospholipide (I) und gegebenenfalls (II) mit einem Innenraum und sphärischer Gestalt ("zwiebelschalenförmiger" Aufbau der Doppelschichten oder Membranen - mulitlamellar). Die Grössenordnung der Liposomen ist variabel zwischen ca. $1{,}0 \times 10^{-8}$ bis ca. $1{,}0 \times 10^{-5}$ m.

Die therapeutische Verwendung von Liposomen als Träger von Wirkstoffen unterschiedlicher Art ist bekannt. Ferner sind Liposomen als Träger von Proteinen, z.B. Antikörpern oder Enzymen, Hormonen, Vitaminen oder Genen oder zu analytischen Zwecken als Träger von markierten Verbindungen vorgeschlagen worden.

Injizierbare Liposomendispersionen sind in dem Uebersichtswerk von Gregoriadis G. (Herausgeber) Liposome Technology, Vol. II, Incorporation of Drugs, Proteins and Genetic Material, CRC Press 1984, beschrieben. Im Uebersichtswerk von Knight, C.G. (Herausgeber), Liposomes: From Physical Structure to Therapeutic Applications, Elsevier 1981, sind im Kapitel 16 auf S. 166 die Vorteile einer solchen Darreichungsform auf Liposomenbasis zusammengefasst.

Der im Zusammenhang mit organischen Resten, z.B. Niederalkyl, Niederalkylen, Nieder-alkoxy, Niederalkanoyl etc., verwendete Ausdruck "Nieder" bedeutet, dass solche organischen Reste, falls nicht ausdrücklich anders definiert, bis einschliesslich 7 und bevorzugt bis einschliesslich 4 Kohlenstoffatome enthalten.

Die Nomenklatur der Phospholipide der Formeln I und II und die Bezifferung der C-Atome erfolgt anhand der in Eur. J. of Biochem. 79, 11 -21 ( 1977) " Nomenclature of Lipids" von der IUPAC-IUB Commission on Biochemical Nomenclature (CBN) gegebenen Empfehlungen (sn-Nomenklatur, stereospecific numbering).

In einem Phospholipid der Formel I sind $R_1$ und $R_2$ mit den Bedeutungen $C_{10-20}$-Acyl vorzugsweise geradkettiges $C_{10-20}$-Alkanoyl mit einer geraden Anzahl an C-Atomen und geradkettiges $C_{10-20}$-Alkenoyl mit einer Doppelbindung und einer geraden Anzahl an C-Atomen.

Geradkettiges $C_{10-20}$-Alkanoyl $R_1$ und $R_2$ mit einer geraden Anzahl an C-Atomen sind beispielsweise n-Dodecanoyl, n-Tetradecanoyl, n-hexadecanoyl oder n-Octadecanoyl.

Geradkettiges $C_{10-20}$-Alkenoyl $R_1$ und $R_2$ mit einer Doppelbindung und einer geraden Anzahl an C-Atomen sind beispielsweise 6-cis-, 6-trans-, 9-cis- oder 9-trans-dodecenoyl, -tetradecenoyl, -hexadecenoyl, -octadecenoyl oder -icosenoyl, insbesondere 9-cis-octadecenoyl (oleoyl).

In einem Phospholipid der Formel I ist n eine ganze Zahl von zwei bis vier, vorzugsweise zwei. Die Gruppe der Formel $-(C_nH_{2n})-$ stellt unverzweigtes oder verzweigtes Alkylen dar, z.B. 1,1-Aethylen, 1,1-, 1,2- oder 1,3-Propylen oder 1,2-, 1,3- oder 1,4-Butylen. Bevorzugt ist 1,2-Aethylen (n=2).

Phospholipide der Formel I sind beispielsweise natürlich vorkommende Kephaline, worin $R_a$, $R_b$ und $R_c$ Wasserstoff bedeuten oder natürlich vorkommende Lecithine, worin $R_a$, $R_b$ und $R_c$ Methyl bedeuten, z.B. Kephalin oder Lecithin aus Sojabohnen, Rinderhirn, Rinderleber oder Hühnerei mit verschiedenen oder identischen Acylgruppen $R_1$ und $R_2$ oder Mischungen davon.

Bevorzugt sind synthetische, im wesentlichen reine Phospholipide der Formel I mit verschiedenen oder identischen Acylgruppen $R_1$ und $R_2$.

Der Begriff "synthetisches" Phospholipid der Formel I definiert Phospholipide, welche bezüglich $R_1$ und $R_2$

eine einheitliche Zusammensetzung haben. Solche synthetischen Phospholipide sind vorzugsweise die weiter vorn definierten Lecithine und Kephaline, deren Acylgruppen $R_1$ und $R_2$ eine definierte Struktur haben und von einer definierten Fettsäure mit einem Reinheitsgrad höher als ca. 95 % abgeleitet sind. $R_1$ und $R_2$ können gleich oder verschieden und ungesättigt oder gesättigt sein. Bevorzugt ist $R_1$ gesättigt, z.B n-Hexadecanoyl, und $R_2$ ungesättigt, z.B. 9-cis-Octadecenoyl (= Oleoyl).

Der Begriff "natürlich vorkommende" Phospholipide der Formel I definiert Phospholipide, welche bezüglich $R_1$ und $R_2$ keine einheitliche Zusammensetzung haben. Solche natürlichen Phospholipide sind ebenfalls Lecithine und Kephaline, deren Acylgruppen $R_1$ und $R_2$ strukturell undefinierbar und von natürlich vorkommenden Fettsäuregemischen abgeleitet sind.

Die Forderung "im wesentlichen reines" Phospholipid definiert einen Reinheitsgrad von mehr als 95 % (Gew.) des Phospholipids (I), welcher anhand geeigneter Bestimmungsmethoden, z.B. papierchromatographisch, nachweisbar ist.

Besonders bevorzugt sind synthetische, im wesentlichen reine Phospholipide der Formel I, worin $R_1$ die Bedeutung geradkettiges $C_{10-20}$-Alkanoyl mit einer geraden Anzahl an C-Atomen und $R_2$ die Bedeutung geradkettiges $C_{10-20}$-Alkenoyl mit einer Doppelbindung und einer geraden Anzahl an C-Atomen haben. $R_a$, $R_b$ und $R_c$ sind Methyl und n ist zwei.

In einem besonders bevorzugten Phospholipid der Formel I bedeuten $R_1$ n-Dodecanoyl, n-Tetradecanoyl, n-Hexadecanoyl oder n-Octadecanoyl und $R_2$ 9-cis-Dodecenoyl, 9-cis-Tetradecenoyl, 9-cis-hexadecenoyl, 9-cis-Octadecenoyl oder 9-cis-Icosenoyl. $R_a$, $R_b$ und $R_c$ sind Methyl und n ist zwei.

Ein ganz besonders bevorzugtes Phospholipid der Formel I ist synthetisches 1-n-Hexadecanoyl-2-(9-cis-octadecenoyl)-3-sn-phosphatidylcholin mit einer Reinheit von mehr als 95 %.

In einem Phospholipid der Formel II sind $R_3$ und $R_4$ mit der gleichen Bedeutung $C_{10-20}$-Acyl wie für $R_1$ und $R_2$ in einem Phospholipid der Formel I definiert.

Die Acylgruppen $R_3$ und $R_4$ können verschieden oder identisch sein

$R_5$ mit der Bedeutung $C_{1-4}$-Alkyl ist beispielsweise Methyl oder Aethyl.

$R_5$ mit den Bedeutungen $C_{1-5}$-Alkyl substituiert durch Carboxy, $C_{2-5}$-Alkyl substituiert durch Hydroxy oder $C_{2-5}$-Alkyl substituiert durch Carboxy oder Hydroxy sind beispielsweise 2-Hydroxyäthyl, 2,3-Dihydroxy-n-propyl, Carboxymethyl, 1- oder 2-Carboxyäthyl, Dicarboxymethyl, 2-Carboxy-2-hydroxyäthyl oder 3-Carboxy-2,3-dihydroxy-n-propyl.

$R_5$ mit der Bedeutung $C_{2-5}$-Alkyl substituiert durch Carboxy und Amino ist z.B. 3-Amino-3-carboxy-n-propyl oder 2-Amino-2-carboxy-n-propyl, vorzugsweise 2-Amino-2-carboxyäthyl. Phospholipide der Formel II mit diesen Gruppen können in Salzform, z.B als Natrium- oder Kaliumsalz, vorliegen.

Bevorzugt sind synthetische, im wesentlichen reine Phospholipide der Formel II mit verschiedenen oder identischen Acylgruppen $R_3$ und $R_4$.

Bezüglich der Definition "synthetisch" und der Forderung "im wesentlichen reines" gelten die weiter vorn genannten, für Phospholipide der Formel I festgelegten Definitionen.

In einem besonders bevorzugten Phospholipid der Formel II aben $R_3$ und $R_4$ die Bedeutung geradkettiges $C_{10-20}$-Alkenoyl mit einer Doppelbindung und einer geraden Anzahl an C-Atomen. $R_5$ ist 2-Amino-2-carboxyäthyl.

In dem bevorzugten Phospholipid der Formel II sind $R_3$ und $R_4$ mit der Bedeutung "$C_{10-20}$-Alkenoyl mit einer Doppelbindung und geraden Anzahl an C-Atomen" vorzugsweise 9-cis-Dodecenoyl, 9-cis-Tetradecenoyl, 9-cis-Hexadecenoyl, 6-cis-Octadecenoyl, 6-trans-Octadecenoyl, 9-cis-Octadecenoyl, 9-trans-Octadecenoyl, 11-cis-Octadecenoyl oder 9-cis-Icosenoyl.

In einem besonders bevorzugten Phospholipid der Formel II haben $R_3$ und $R_4$ identische Bedeutungen, z.B. 9-cis-Dodecenoyl, 9-cis-Tetradecenoyl, 9-cis-Hexadecenoyl, 9-cis-Octadecenoyl oder 9-cis-Icosenoyl.

Ein ganz besonders bevorzugtes Phospholipid der Formel II ist synthetisches Natrium-1,2-di-(9-cis-octadecenoyl)-3-sn-phosphatidyl-S-serin mit einer Reinheit von mehr als 95 %.

Für die Acylreste in den Phospholipiden der Formeln I und II sind auch die in Klammern angegebenen Bezeichnungen gebräuchlich:

9-cis-Dodecenoyl (Lauroleoyl), 9-cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 11-cis-Octadecenoyl (Vaccenoyl), 9-cis-Icosenoyl (Gadoleoyl), n-Dodecanoyl (Lauroyl), n-Tetradecanoyl (Myristoyl), n-Hexadecanoyl (Palmitoyl), n-Octadecanoyl (Stearoyl), n-Icosanoyl (Arachidoyl).

Als injizierbarer Wirkstoff oder Wirkstoffkombination eignen sich in erster Linie durch Injektionslösungen verabreichbare, wasserlösliche, aber auch schwerlösliche, gegebenenfalls kristalline, lipophile Wirkstoffe.

Schwerlösliche Wirkstoffe liegen z.B. als wasserlösliche, pharmazeutisch annehmbare Salze vor, z.B. als

Hydrobromid, Hydrochlorid, Mesylat, Acetat, Succinat, Lactat, Tartrat, Fumarat, Sulfat, Maleat, etc.

Geeignete pharmazeutische Wirkstoffe sind z.B. entzündungshemmende Mittel, z.B. Indomethacin, Acetylsalicylsäure, Ketoprofen, Ibuprofen, Mefenaminsäure, Dexamethason, Natriumdexamethasonsulfat, Hydrocortison oder Prednisolon, Coronardilatatoren, z.B. Nifedipin, Isosorbiddinitrat, Nitroglycerin, Diltiazem, Trapidil, Dipyridamol oder Dilazep, Prostaglandine, z.B. Prostaglandin $E_1$, $E_2$ oder $F_{2\alpha}$, periphere Vasodilatatoren, z.B. Ifenprodil, Cinepazetmaleat, Cyclandelat, Cinnarizin oder Pentoxyphyllin, Antibiotica, z.B. Ampicillin, Amoxycillin, Cephalexin, Cefradin, Cefroxadin, Cefaclor, Erythromycin, Bacampicillin, Minocyclin oder Chloramphenicol, krampflösende Mittel, z.B. Propanthelin, Atropin oder Scopolamin, Antitussiva und Antiasthmatica, z.B. Theophyllin, Aminophyllin, Methylephedrin, Procatechol, Trimethoquinol, Codein, Clofedanol oder Dextromethorphan, Diuretica, z.B. Furosemid oder Acetazolamid, muskelentspannende Mittel, z.B. Chlorphenesincarbamat, Tolperison, Eperison oder Baclofen, schwache Beruhigungsmittel, z.B. Oxazolam, Diazepam, Clotiazepam, Medazepam, Temazepam oder Fludiazepam, starke Beruhigungsmittel, z.B. Sulpirid, Clocapramin oder Zotepin, Beta-Blocker, z.B. Pindolol, Propranolol, Carteolol, Oxprenolol, Metoprolol oder Labetalol, Antiarrhythmica, z.B. Procainamid, Disopyramid, Ajimalin oder Quinidin, Antigichtmittel wie Allopurinol, Anticoagulanzien wie Ticlopidin, Antiepileptica, z.B. Phenytoin, Valproat oder Carbamazepin, Antihistaminica, z.B. Chlorpheniramin, Clemastin, Mequitazin, Alimemazin, Cyproheptadin, Mittel gegen Uebelkeit und Schwindel, z.B. Diphenidol, Metoclopramid, Domperidon oder Bethahistin, Blutdrucksenkende Mittel, z.B. Reserpin, Rescinnamin, Methyldopa, Prazosin, Clonidin oder Budralazin, Sympathomimetica, z.B. Dihydroergotamin, Isoproterenol oder Etilefrin, Expectoranzien, z.B. Bromhexin, Carbocystein, L-Ethylcystein oder L-Methylcystein, orale Antidiabetica, z.B. Glibenclamid oder Tolbutamid, kardiovaskuläre Mittel, z.B. Ubidecarenon oder Adenosin.

Bevorzugt sind Antihypercalcämika aus der Calcitoninreihe, z.B. synthetisch herstellbares Salm-, Human- und Schweinecalcitonin sowie das Aalcalcitoninpräparat, z.B. 1,7-Asu-Aal-Calcitonin (Elcatonin), oder lipophile oder hydrophile Immunmodulatoren aus der Muramylpeptidreihe, z.B. N-Acetyl-D-muramyl-L-alanyl-D-isoglutaminyl-L-alanin-2-(1,2-dipalmitoyl-sn-glycero-3-hydroxy-phosphoryloxy)-ethylmid, N-Acetyl-D-muramyl-L-alanyl-D-glutaminsäure-($C^{\gamma}$-L-alanin-2-( 1,2-dipalmitoyl-sn-glycero-3-hydroxy-phosphoryloxy)-ethylamid)-dinatriumsalz, N-Acetyl-D-muramyl-L-alanyl-D-isoglutamin-natriumsalz, N-Acetyldesmethyl-muramyl-L-alanyl-D-isoglutamin-natriumsalz, N-Acetyl-D-muramyl-L-alanyl-D-glutamin-$\alpha$-n-butylester, $N^{\alpha}$-(N-acetyl-D-muramyl-L-alanyl-D-isoglutaminyl)-$N^{\gamma}$-stearoyl-L-lysin oder 6-O-stearoyl-N-acetyl-D-muramyl-L-alanyl-D-isoglutamin.

Die pharmazeutisch annehmbare Trägerflüssigkeit d) ist Wasser, das nach den Vorschritten der nationalen Pharmakopöen tür intravenöse Lösungen keim- und pyrogenfrei gemacht wurde.

In der Trägerflüssigkeit d) sind die Komponenten a), b) und c) oder a) und c) als Liposomen so enthalten, dass sich mehrere Tage bis Wochen keine Feststoffe oder feste Aggregate wie Mizellen zurückbilden und die klare oder gegebenenfalls leicht opaleszierende Flüssigkeit mit den genannten Komponenten, gegebenenfalls nach Filtration, als Injektionslösung vorzugsweise intravenös, applizierbar ist.

In der Trägerflüssigkeit d) können für Injektionszubereitungen verwendbare, nicht toxische Hilfsstoffe enthalten sein, z.B. wasserlösliche Hilfsstoffe welche zur Herstellung von isotonischen Bedingungen erforderlich sind, z.B. ionische Zusätze wie kochsalz oder nichtionische Zusätze (Gerüstbildner) wie Sorbit, Mannit oder Glucose oder wasserlösliche Stabilisatoren für die Liposomendispersion wie Lactose, Fructose oder Sucrose. Insbesondere sind diese Zusätze, z.B. Kochsalz oder Mannit, in den vorgeschriebenen Mengen enthalten, welche zur Herstellung von isotonischen Bedingungen der Injektionslösungen erforderlich sind.

Zusätzlich zu den wasserlöslichen Hilfsstoffen können in der Trägerflüssigkeit für flüssige pharmazeutische Formulierungen verwendbare Emulgatoren, Netzmittel oder Tenside vorhanden sein, insbesondere Emulgatoren wie Ölsäure, nichtionische Tenside vom Typ Fettsäure-Polyhydroxyalkoholester wie Sorbitanmonolaurat, -oleat, -stearat oder -palmitat, Sorbitantristearat oder -trioleat, Polyoxyethylen-Addukte von Fettsäure-Polyhydroxyalkoholestern wie Polyoxyethylen-sorbitanmonolaurat, -oleat, -stearat, -palmitat, -tristearat oder -trioleat, Polyethylenglycol-Fettsäureester wie Polyoxyethylstearat, Polyethylenglycol-400-stearat, Polyethylenglycol-2000-stearat, insbesondere Ethylenoxid-Propylenoxid Blockpolymere vom Typ Pluronic® (Wyandotte Chem. Corp.) oder Synperonic® (ICI).

Konzentrierte Essigsäure hat einen Gehalt von mehr als 90 % (Gew.), wobei die restlichen Gewichtsanteile auf Wasser entfallen. Bevorzugt ist konzentrierte gereinigte Essigsäure mit einem Gehalt von mehr als 95 %, insbesondere mehr als 99 %, welche unter dem Trivialnamen Eisessig bekannt ist.

In Abhängigkeit von der Löslichkeit der Komponenten a) und c) oder a), b) und c) in der konzentrierten Essigsäure bildet sich eine Lösung. Diese wird anschliessend in der Trägerflüssigkeit d), der man für Injektionszubereitungen geeignete Hilfsstoffe gegebenenfalls zugesetzt hat, dispergiert.

Die Dispersion selbst erfolgt beispielsweise durch Schütteln (z.B. Vortex-Mischer) oder Rühren der Trägerflüssigkeit. Die Bildung von Liposomen, welche gross, klein, unilamellar oder multilamellar sein können, findet spontan, d.h. ohne zusätzliche Energiezufuhr von aussen und mit grosser Geschwindigkeit statt. Es können ca. 0, 1 bis 50 Gewichtsprozent (bezogen auf das Gesamtgewicht der wässrigen Dispersion), vorzugsweise 2

bis 20 Gewichtsprozent, der Essigsäurelösung oder -dispersion in wässriger Phase dispergiert werden.

Ebenfalls kann man die genannten Komponenten mit einem Hochdruckhomogenisator, z.B. wie in Pharm. Ind. 52, Nr. 3 (1990) auf den Seiten 343-347 beschrieben, dispergieren und dadurch eine besonders einheitliche Liposomendispersion herstellen.

Man dispergiert bei Temperaturen unterhalb ca. 36°C, vorzugsweise bei Raumtemperatur. Gegebenenfalls führt man das Verfahren unter Kühlen und/oder Inertgasatmosphäre, z.B. Stickstoff- oder Argonatmosphäre, durch. Die erhältlichen Liposomen sind in wässriger Phase sehr lange (bis zu mehrere Wochen oder Monate) beständig.

Die Grösse der gebildeten Liposomen ist u.a. von der Menge des Wirkstoffs und der Lipidkomponenten, deren Mischungsverhältnis und konzentration in der wässrigen Dispersion abhängig. So kann man durch Erhöhung oder Erniedrigung der Konzentration der einzelnen Lipidkomponenten wässrige Phasen mit einem hohen Anteil an kleinen oder grossen Liposomen herstellen.

Die Grösse und Struktur (multilamellar - unilamellar) der gebildeten Liposomen ist auch von der Wahl des betreffenden Verfahrens abhängig. Bei Schütteln oder Rühren, z.B. mit konventionellen Rührern mit Propeller oder Flügelblatt oder mit einem Magnetrührer, erhält man Dispersionen mit einem hohen Anteil an grossen multilamellaren Liposomen. Die Erhöhung der Rührfrequenz oder der Uebergang zu Phasenmischern mit hohen Scherkräften bewirkt eine Erhöhung des Anteils an kleinen, multilamellaren Liposomen. Die Behandlung mit Ultraschall ergibt einen hohen Anteil an unilamellaren Liposomen in der Dispersion.

Man puffert vorzugsweise sauer reagierende wässrige Dispersionen auf pH 7,0 bis 7,8, vorzugsweise 7,2 bis 7,4, ab. Dafür lassen sich vorzugsweise pharmazeutisch annehmbare Pufferlösungen verwenden, deren Herstellung in diversen nationalen Pharmakopöen, z.B. der Europäischen, U.S., Deutschen oder Britischen Pharmakopöe, beschrieben ist. Man kann die Dispersion auch durch Zugabe einer pharmazeutisch annehmbaren, verdünnten wässrigen Base neutralisieren, z.B. verdünnter Natronlauge. Man neutralisiert üblicherweise unter gleichzeitiger pH-Kontrolle. Gegebenenfalls füllt man mit sterilem, keimfreiem und pyrogenfreiem Wasser auf das erforderliche Injektionsvolumen auf. Die Injektionszubereitung ist direkt, z. B. subkutan, vorzugsweise intravenös, applizierbar.

Durch Nachbehandlung der Liposomendispersion, z.B. durch Beschallung mit Ultraschall oder Extrusion durch geradporige Filter (z.B. Nucleopore®) kann man eine besonders einheitliche Grössenverteilung der Liposomen erhalten.

Die Trennung und Isolierung einer Fraktion von grossen Liposomen von einer Fraktion mit kleinen Liposomen, sofern überhaupt notwendig, erfolgt mittels herkömmlicher Trennmethoden, z.B. Gelfiltration oder Ultrafiltration, z.B. mit Sepharose®4B oder Sephacryl® (Pharmacia SE) als Träger, oder durch Sedimentation der Liposomen in der Ultrazentrifuge, z.B. mit einem Schwerefeld bei 160,000 x g. Beispielsweise setzen sich nach mehrstündigem, z.B. ca. dreistündigem, Zentrifugieren in diesem Schwerefeld Liposomen ab, während die kleinen Liposomen dispergiert bleiben und dekantiert werden können. Nach mehrmaligem Zentrifugieren erreicht man eine vollständige Trennung der grossen von den kleinen Liposomen.

Insbesondere durch Gelfiltration kann man alle in der wässrigen Phase befindlichen Liposomen mit einem Durchmesser grösser als ca. $6,0 \times 10^{-8}$ m sowie nicht verkapselte Komponenten und überschüssige, dispergierte Lipide, welche in hochmolekularen Aggregaten vorliegen, abtrennen und so eine wässrige Dispersion mit einer Fraktion von Liposomen mit relativ einheitlicher Grösse herstellen.

Die erfolgte Bildung von Liposomen und ihr Gehalt in wässriger Phase lässt sich in an sich bekannter Weise anhand verschiedener physikalischer Messmethoden nachweisen, z.B. mit gefriergebrochenen (freeze fracture) Proben und Dünnschnitten im Elektronenmikroskop oder durch Röntgendiffraktion, durch dynamische Lichtstreuung, durch Massenbestimmung des Filtrates in der analytischen Ultrazentrifuge und vor allem spektroskopisch, z.B. im Kernresonanzspektrum ($^{1}H$, $^{13}C$ und $^{31}P$).

Die Liposomendispersion ist direkt applizierbar, kann aber auch durch Gefriertrocknen in ein Lyophilisat überführt werden, das man unmittelbar vor der Applikation durch Zugabe von Wasser mit dem vorgesehenen Injektionsvolumen rekonstituiert.

Die Erfindung betrifft in erster Linie ein Verfahren zur Herstellung einer intravenös applizierbaren Liposomendispersion enthaltend

a) synthetisches, im wesentlichen reines 1-n-Hexadecanoyl-2-(9-cis-octadecenoyl)-3-sn-phosphatidylcholin (I), gegebenenfalls kombiniert mit

b) synthetischem, im wesentlichen reinem Natrium-1,2-di-(9-cis-octadecenoyl)-3-sn-phosphatidyl-S-serin (II),

c) den intravenös zu applizierenden Wirkstoff und

d) eine pharmazeutisch annehmbare Trägerflüssigkeit und gegebenenfalls für Injektionszubereitungen geeignete hilfsstoffe.

Die Erfindung betrifft vor allem ein Verfahren zur herstellung einer intravenös applizierbaren Liposomendispersion enthaltend

a) synthetisches, im wesentlichen reines 1-n-Hexadecanoyl-2-(9-cis-octadecenoyl)-3-sn-phosphatidylcholin (I), gegebenenfalls kombiniert mit

b) synthetischem, im wesentlichen reinem Natrium-1,2-di-(9-cis-octadecenoyl)-3-sn-phosphatidyl-S-serin (II),

c) Carbamazepin, synthetisches Humancalcitonin oder N-Acetyl-D-muramyl-L-alanyl-D-isoglutaminyl-L-alanin-2-(1,2-dipalmitoyl-sn-glycero-3-hydroxyphosphoryloxy)-äthyl-amid und

d) eine pharmazeutisch annehmbare Trägerflüssigkeit und gegebenenfalls für Injektionszubereitungen geeignete Hilfsstoffe.

Die folgenden Beispiele illustrieren die Erfindung.

Beispiel 1:

In einem Rundkolben werden 250 mg einer Phospholipidmischung bestehend aus zu mindestens 95 % reinem 1-n-Hexadecanoyl-2-(9-cis-octadecenoyl)-3-sn-phosphatidylcholin und zu mindestens 95 % reinem 1,2-Di-(9-cis-octadecenoyl)-3-sn-phosphatidyl-S-serin in einem Gewichtsverhältnis von 7:3 und 1 mg N-Acetyl-D-muramyl-L-alanyl-D-isoglutaminyl-L-alanin-2-( 1,2-dipalmitoyl-sn-glycero -3 -hydroxy-phosphoryloxy)-äthylamid in 0,25 ml Eisessig gelöst. Unter Rühren gibt man die äquimolare Menge 2 N NaOH-Lösung (= 2,075 ml) hinzu. Dabei bildet sich eine Liposomendispersion, welche mit einer geeigneten Pufferlösung (pH = 7,2) auf das gewünschte Volumen (50 ml) aufgefüllt wird. Die Pufferlösung wird so ausgewählt, dass man einen pH-Wert der Dispersion von ca. 7,2 bis 7,4 erhält und eine Osmolarität von ca. 290 mosmol resultiert. Vor der Applikation kann man die Dispersion in einem Autoklaven keimfrei machen.

Beispiel 2:

In einer Glasviale werden 80 mg zu mindestens 95 % reines 1-n-Hexadecanoyl-2-lyso-3-sn-phosphatidylcholin, 40 mg zu mindestens 95 % reines 1,2-Di-(9-cis-octadecenoyl)-3-sn-phosphatidyläthanolamin und 80 mg Oelsäure zusammen mit 15 mg Carbamazepin eingewogen. Diese Mischung wird unter Rühren in 0,5 ml Eisessig gelöst. Unter Rühren gibt man eine äquimolare Menge 2 N NaOH-Lösung (4,15 ml) hinzu. Dabei bildet sich eine Liposomendispersion. Die weiteren Schritte werden analog Beispiel 1 vorgenommen.

Beispiel 3:

In einem Rundkolben werden 250 mg des Phospholipidgemisches gemäss Beispiel 1 in 250 μl Eisessig enthaltend 310 μg synthetisches humancalcitonin gelöst. Diese Lösung wird unter Rühren mit 2,08 ml sterilfiltrierter 2 N NaOh-Lösung versetzt und mit einem Vortex-Gerät auf Stufe 10 homogenisiert. Die gebildete Liposomendispersion wird durch Zugabe von keimfreiem, pyrogenfreiem Wasser zur Injektion auf ein Volumen von 20 ml verdünnt. Diese Dispersion eignet sich für die intramuskuläre Applikation.

## Patentansprüche

**1.** Verfahren zur Herstellung einer injizierbaren Liposomendispersion enthaltend

a) ein Phospholipid der Formel

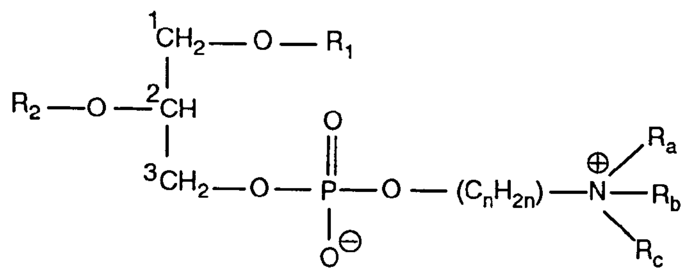

(I),

worin $R_1$ $C_{10-20}$-Acyl, $R_2$ Wasserstoff oder $C_{10-20}$-Acyl, $R_a$, $R_b$ und $R_c$ Wasserstoff oder $C_{1-4}$-Alkyl und n eine ganze Zahl von eins bis vier darstellen, gegebenenfalls kombiniert mit einem weiteren

b) Phospholipid der Formel

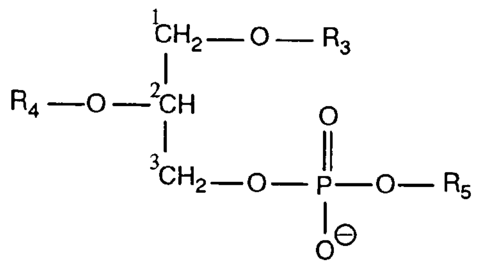

(II),

worin $R_3$ $C_{10-20}$-Acyl, $R_4$ Wasserstoff oder $C_{10-20}$-Acyl und $R_5$ Wasserstoff, $C_{1-4}$-Alkyl, $C_{1-5}$-Alkyl substituiert durch Carboxy, $C_{2-5}$-Alkyl substituiert durch Hydroxy, $C_{2-5}$-Alkyl substituiert durch Carboxy und Hydroxy oder $C_{2-5}$-Alkyl substituiert durch Carboxy und Amino,

c) den zu injizierenden Wirkstoff oder eine Wirkstoffkombination und

d) eine pharmazeutisch annehmbare Trägerflüssigkeit und gegebenenfalls weitere für Injektionszubereitungen geeignete Hilfsstoffe, dadurch gekennzeichnet, dass man eine Lösung oder Suspension der Komponenten a) und c) oder a), b) und c) in konzentrierter Essigsäure in der Trägerflüssigkeit d) dispergiert und die erhältliche Dispersion auf einen physiologisch annehmbaren pH-Wert bringt und gegebenenfalls für Injektionszubereitungen geeignete Hilfsstoffe zusetzt und gegebenenfalls eine Fraktion von Liposomen mit einem gewünschten Bereich des Durchmessers abtrennt.

**2.** Verfahren gemäss Anspruch 1 zur Herstellung einer intravenös applizierbaren Liposomendispersion enthaltend

a) synthetisches, im wesentlichen reines 1-n-Hexadecanoyl-2-(9-cis-octadecenoyl)-3-sn-phosphatidylcholin (I), gegebenenfalls kombiniert mit

b) synthetischem, im wesentlichen reinem Natrium-1,2-di-(9-cis-octadecenoyl)-3-sn-phosphatidyl-S-serin (II),

c) den intravenös zu applizierenden Wirkstoff und

d) eine pharmazeutisch annehmbare Trägerflüssigkeit und gegebenenfalls für Injektionszubereitungen geeignete Hilfsstoffe, dadurch gekennzeichnet, dass man die in Anspruch 1 genannten Massnahmen durchführt.

**3.** Verfahren gemäss Anspruch 1 zur Herstellung einer intravenös applizierbaren Liposomendispersion enthaltend

a) synthetisches, im wesentlichen reines 1-n-Hexadecanoyl-2-(9-cis-octadecenoyl)-3-sn-phosphatidylcholin (I), gegebenenfalls kombiniert mit

b) synthetischem, im wesentlichen reinem Natrium- 1,2-di-(9-cis-octadecenoyl)-3-sn-phosphatidyl-S-serin (II),

c) Carbamazepin, synthetisches Humancalcitonin oder N-Acetyl-D-muramyl-L-alanyl-D-isoglutaminyl-L-alanin-2-( 1,2-dipalmitoyl-sn-glycero-3-hydroxyphosphoryloxy)-äthylamid und

d) eine pharmazeutisch annehmbare Trägerflüssigkeit und gegebenenfalls für Injektionszubereitungen geeignete Hilfsstoffe, dadurch gekennzeichnet, dass man die in Anspruch 1 genannten Massnahmen durchführt.

**4.** Verfahren gemäss einem der Ansprüche 1 bis 3, dadurch gekennzeichnet, dass man eine Lösung oder Suspension der Komponenten a) und c) oder a), b) und c) in Eisessig in der Trägerflüssigkeit d) dispergiert und die erhältliche Dispersion auf den pH-Wert von 7,2 bis 7,4 bringt.

**5.** Die nach dem Verfahren gemäss Anspruch 1 erhältliche Liposomendispersion.